**Europäisches Patentamt**

**European Patent Office**

**Office européen des brevets**

(19)

(11) Veröffentlichungsnummer: **0 038 525**
**B1**

(12) **EUROPÄISCHE PATENTSCHRIFT**

(45) Veröffentlichungstag der Patentschrift:
31.08.83

(21) Anmeldenummer: 81102876.0

(22) Anmeldetag: 15.04.81

(51) Int. Cl.³: **A 61 B 3/12,** A 61 B 3/10,
G 02 B 27/32

(54) Augenrefraktometer.

(30) Priorität: 18.04.80 DE 3014907

(43) Veröffentlichungstag der Anmeldung:
28.10.81 Patentblatt 81/43

(45) Bekanntmachung des Hinweises auf die Patenterteilung:
31.08.83 Patentblatt 83/35

(84) Benannte Vertragsstaaten:
AT CH FR GB IT LI NL

(56) Entgegenhaltungen:
DE-A-1 955 859
DE-B-1 267 877
DE-C-205 177
DE-C-450 578
FR-A-1 149 085
FR-A-2 069 843
FR-A-2 365 325
GB-A-2 005 436
US-A-3 824 005
US-A-3 830 562

(73) Patentinhaber: **Firma Carl Zeiss, Postfach 1369/1380,**
**D-7082 Oberkochen (DE)**

(72) Erfinder: **Hanemann, Gerhard, Jenaer Strasse 4,**
**D-7082 Oberkochen (DE)**
Erfinder: **Muchel, Franz, Dipl.-Phys, Lortzingstrasse 2,**
**D-7923 Königsbronn (DE)**
Erfinder: **Schulz, Kurt, Mozartweg 20,**
**D-7082 Oberkochen (DE)**
Erfinder: **Vogel, Albrecht, Hainbuchenweg 14,**
**D-7082 Oberkochen (DE)**

## Augenrefraktometer

Die Erfindung betrifft ein Augenrefraktometer zur objektiven Refraktionsbestimmung des Auges mit einem Optometersystem zur Abbildung einer Testmarke auf die Netzhaut des Prüflings, mit einem Beobachtungssystem für die Testmarke und einem Visiersystem zur Ausrichtung des Refraktometers zum Patientenauge.

Mit derartigen Augenrefraktometern werden die sphärischen und zylindrischen Wirkungen des zu verordnenden Brillenglases und seine Achsenlage bestimmt. Vom Benutzer wird vor allem ein sicheres, schnelles und ermüdungsfreies Arbeiten mit dem Augenrefraktometer verlangt.

Aus dem Prospekt 369 – 2d/4 – 2.75 Mehd. der Firma Rodenstock ist ein Augenrefraktometer bekannt, das diese Forderungen teilweise erfüllt. Nachteilig bei dem bekannten Augenrefraktometer ist jedoch die Tatsache, dass das Gerät räumlich soweit voneinander getrennte Einblicksokulare für die Entfernungsmesserskala und für die Beobachtung von Testfigur und Skalenablesung hat, dass während der Messung ein wiederholter Positionswechsel des Beobachters erforderlich ist. Ausserdem sind die Bedienelemente bei diesem bekannten Gerät nicht optimal angeordnet. Es besitzt nämlich (vom Beobachter aus gesehen) an seiner rechten Seite einen Drehknopf für Dioptrienverstellung, an seiner linken Seite einen Drehknopf für die Einstellung der Hauptschnittlage. Da die Messung mit dem Augenrefraktometer in vivo ein dynamischer Vorgang ist (d.h., die Gerätepupille muss während des Messvorgangs wiederholt zur Augenpupille des Probanden nachgeführt werden) muss der Beobachter, dessen eine Hand an einem Koordinaten-Steuerhebel zur Verstellung des Gerätes verbleiben muss, beim optischen Messvorgang mehrmals von der einen Geräteseite zur anderen umgreifen.

Der Erfindung liegt deshalb die Aufgabe zugrunde, ein Augenrefraktometer zu schaffen, das ein sichereres, schnelleres und ermüdungsfreieres Arbeiten als das bekannte Gerät gestattet.

Diese Aufgabe wird erfindungsgemäss dadurch gelöst, dass im Abbildungsstrahlengang ein lotrecht verschiebbares Abbildungsobjektiv vorgesehen ist, das die Testfigur in eine Zwischenbildebene abbildet, dass ein Strahlungsteilungsprisma für die geometrische Teilung des Abbildungs- und des Beobachtungsstrahlengangs vorgesehen ist, dass ein dem Abbildungs- und dem Beobachtungsstrahlengang gemeinsames Ophthalmoskopobjektiv vorgesehen ist, welches das Zwischenbild der Testfigur auf die Netzhaut des Probanden projiziert und von dort wieder in der Zwischenbildebene abbildet, dass ein mit dem Abbildungsobjektiv synchron verschiebbares Beobachtungsobjektiv vorgesehen ist, welches das Netzhautzwischenbild der Testfigur nach Reflexion an dem Strahlenteilungsprisma in die Okularebene des rechten Tubuseinblicks eines Binokulartubus abbildet, dass im Visiersystem ein Visierobjektiv vorgesehen ist, das die Iris und die Augenpupille des Probanden in die Skalenebene eines Entfernungsmessers abbildet, dass sich in dieser Skalenebene eine Skala für Dioptrienanzeige und eine Skala für die Achsenlage befindet und dass zur Beobachtung der Skalen das linke Okular des binokularen Tubus vorgesehen ist, dass weiter für die Dioptrieneinstellung und für die Hauptschnittlageneinstellung koaxial zueinander angeordnete Bedienelemente vorgesehen sind, und dass ein Hebel mit drei Raststellungen für die Betätigung eines Blendenmechanismus für die Skalen- und Testfigurenbetrachtung vorgesehen ist.

Vorteilhafterweise sind die Bedienelemente und der Hebel in doppelter Ausführung vorgesehen und zu beiden Seiten des Gerätes angebracht.

In einem zweckmässigen Ausführungsbeispiel ist für die Messskalen eine digitale Anzeigevorrichtung vorgesehen. Die mit der Erfindung erzielten Vorteile bestehen insbesondere darin, dass durch den binokularen Einblick die gesamte Untersuchung in einem Beobachtungsgang und ohne jeden Positionswechsel der beteiligten Personen erfolgen kann. Der Hornhautscheitelabstand ist zwischen 10 und 18 mm vorwählbar und wird entsprechend der Augenlage des Probanden oder passend zur modischen Brillenfassung bestimmt. Durch die koaxial angeordneten Drehknöpfe zur Ermittlung der Dioptrienwerte und der Achsenlage wird wiederholtes Umgreifen während der Messungen vermieden.

Ein Ausführungsbeispiel der Erfindung ist in der Zeichnung dargestellt und wird im folgenden näher beschrieben. Es zeigen

Fig. 1 das erfindungsgemässe Augenrefraktometer in perspektivischer Ansicht;

Fig. 2 den optischen Strahlengang in dem in Fig. 1 dargestellten Augenrefraktometer in schematischer Darstellung.

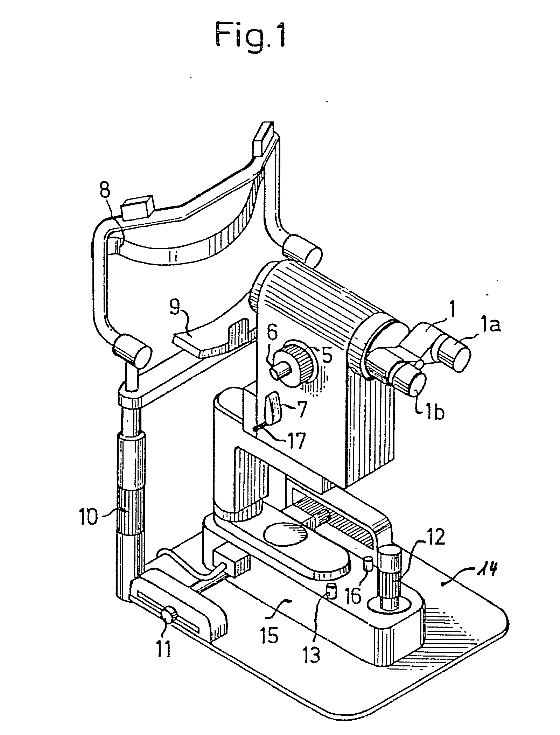

In Fig. 1 ist mit 1 der binokulare Beobachtungstubus mit den Okularen 1a und 1b bezeichnet. Mit 6 und 5 sind die – vom Benutzer her gesehen – auf der linken Geräteseite angeordneten koaxialen Bedienelemente für die Dioptrieneinstellung und die Hauptschnittlageneinstellung gekennzeichnet. Mit 7 ist ein auf der linken Seite sichtbarer Schalthebel bezeichnet, der drei Raststellen aufweist. In der ersten ist im Okular 1b eine Skala für den Hornhautscheitelabstand und ein Bild der Iris sowie der Augenpupille des Probandenauges sichtbar, in der zweiten Raststellung sind im Okular 1b die Anzeigenskalen für Dioptrien und Achsenlage gemeinsam sichtbar und in der dritten Raststellung ist das Sehfeld im Okular 1b abgedeckt. Mit 8 und 9 sind Stirnstütze und Kinnauflage für den Probanden bezeichnet. 10 ist der in Höhe verstellbare Träger für die Kinnauflage. Mit der Klemmschraube 11 kann die Einstellung für den Hornhautscheitelabstand (HSA) fixiert werden. Mit 12 ist ein ansich bekannter Koordinatensteuerhebel bezeichnet, mit dessen Hilfe das Augenrefraktometer in allen drei Koordinaten bewegt werden kann. Die Klemmschraube 13 dient zur Fixierung der Instrumentenbasis 15 auf der Grundplatte 14.

Ausserdem ist in 16 ein Helligkeitsregler für die Beleuchtung und in 17 ein in den Strahlengang einklappbares Rotfilter vorgesehen. Die Bedienelemente 5, 6, 7 sind auch auf der rechten Geräteseite vorgesehen.

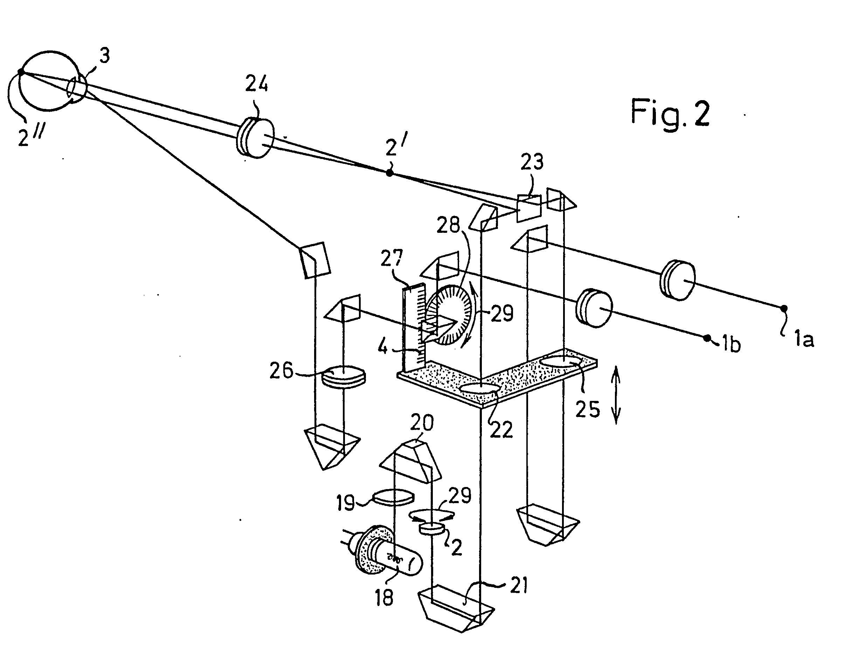

In der Fig. 2 ist die zur Beleuchtung und Abbildung der Testfigur 2 vorgesehene Halogenlampe mit 18 und der Kollektor mit 19 bezeichnet. Die Prismen 20, 21 dienen zur Lichtumlenkung. Das lotrecht verschiebbare Abbildungsobjektiv 22 bildet die Testfigur 2 in die Zwischenbildebene 2' ab. Das Ophthalmoskopobjektiv 24 projiziert das Zwischenbild 2' auf die Netzhaut 2'' des Probanden. Im selben Strahlenverlauf wird die genau definierte Austrittspupille der Beleuchtung in die zur Hälfte benutzte Augenpupille des Probanden abgebildet. Im Beobachtungsstrahlengang der mittels des Strahlenteilungsprismas 23 geometrisch vom Abbildungsstrahlengang getrennt wird, wird das Netzhautbild der Testfigur 2'' von dem Ophthalmoskopobjektiv 24 aufgenommen und erscheint wieder in der Zwischenbildebene 2'. Nach Reflexion an dem Strahlenteilungsprisma 23 bildet das Beobachtungsobjektiv 25, welches synchron mit dem Objektiv 22 in Pfeilrichtung verschiebbar ist, das Netzhautzwischenbild der Testfigur in die Okularebene 1a des rechten Tubuseinblicks ab.

Zum Einstellen des Hornhautscheitelabstandes (HSA) und zum Ablesen der Dioptrien- und der Taboskala bildet das Visierobjektiv 26 die Iris und die Augenpupille 3 des Probanden in die Skalenebene 4 des Entfernungsmessers ab. In der gleichen Ebene befinden sich die Skala 27 für die Dioptrienanzeige und die Skala 28 für die Hauptschnittlage (Taboskala). Die Beobachtung der Skalen erfolgt durch das linke Okular 1b des binokularen Tubus. Testfigur 2 und Taboskala 28 sind gleichzeitig in der Drehrichtung 29 verstellbar.

Das Augenrefraktometer ermöglicht also die Ablesung des vorgewählten, definierten Abstandes zwischen augennahem Brillenglasscheitel und Hornhautscheitel des Probanden («HSA-Einstellung»). Weiterhin ermöglicht das linke Okular in einer anderen Raststellung des Hebels 7 die Ablesung der beispielsweise digital angezeigten Messskalen (Dioptrien- und Hauptschnittskala) für den vorgewählten Hornhautscheitelabstand. Durch das rechte Okular 1a wird vom Untersuchenden unabhängig von der Stellung des Hebels 7 das Netzhautbild 2'' der Testfigur 2 beobachtet. Die Seheindrücke vom rechten Okular 1a und vom linken Okular 1b verschmelzen bei der Beobachtung zu einem visuellen Bild.

Mit dem in Fig. 1 mit 5 bezeichneten grossen Drehknopf wird die Dioptrienverstellung, mit dem mit 6 bezeichneten kleinen Drehknopf die Hauptschnittlageneinstellung bewirkt. Mit dem Schalthebel 7 ist in der auf den Beobachter zugerichteten ersten Raststellung im linken Okular die Entfernungsmesserskala (HSA-Einstellung) sichtbar. Gleichzeitig wird die Abbildung eines runden Leuchtfeldes als Justierhilfe für die Zentrierung «Austrittspupille der Beleuchtung zur Probandenpupille» auf der Iris des Prüflingsauges beobachtet. Wenn der Hebel 7 in mittlerer Raststellung gebracht ist, wird durch einen hier nicht dargestellten Blendenmechanismus die Entfernungsmesserskala abgedeckt und die beiden Skalen 27 und 28 im linken Okular freigegeben. Die beiden entsprechenden Skalenfenster sind beleuchtet. Bei der dritten Raststellung des Schalthebels 7, die vom Beobachter weg gerichtet ist, deckt der erwähnte Blendenmechanismus das Sehfeld des linken Okulars vollständig ab; die Beleuchtungsfunktionen der obengenannten Hebelstellungen sind ausgeschaltet. Sichtbar ist dann nur im rechten Okular die Abbildung des Netzhautbildes der Testfigur. Durch dieses Messen ohne Darbietung von Skalen soll eine Beeinflussung der Wiederholeinstellungen vermieden werden.

Bei jeder Stellung des Schalthebels 7 ist im rechten Okular 1a das Netzhautbild 2'' der Testmarke 2 sichtbar.

**Patentansprüche**

1. Augenrefraktometer zur objektiven Refraktionsbestimmung des Auges mit einem Optometersystem zur Abbildung einer Testmarke auf der Netzhaut des Probanden, mit einem Beobachtungssystem für die Testmarke und einem Visiersystem zur Ausrichtung des Refraktometers zum Probandenauge, dadurch gekennzeichnet, dass im Abbildungsstrahlengang (30) ein lotrecht verschiebbares Abbildungsobjektiv (22) vorgesehen ist, das die Testfigur (2) in eine Zwischenbildebene (2') abbildet, dass ein Strahlungsteilungsprisma (23) für die geometrische Teilung des Abbildungs- und des Beobachtungsstrahlengangs vorgesehen ist, dass ein dem Abbildungs- und dem Beobachtungsstrahlengang gemeinsames Ophthalmoskopobjektiv (24) vorgesehen ist, welches das Zwischenbild (2') der Testfigur (2) auf die Netzhaut (2'') des Probanden projiziert und von dort wieder in der Zwischenbildebene (2') abbildet, dass ein mit dem Abbildungsobjektiv (22) synchron verschiebbares Beobachtungsobjektiv (25) vorgesehen ist, welches das Netzhautzwischenbild (2') der Testfigur nach Reflexion an dem Strahlenteilungsprisma (23) in die Okularebene (1a) des rechten Tubuseinblicks eines Binokulartubus abbildet, dass im Visiersystem ein Visierobjektiv (26) vorgesehen ist, das die Iris und die Augenpupille des Probanden in die Skalenebene (4) eines Entfernungsmessers abbildet, dass sich in dieser Skalenebene eine Skala (27) für Dioptrienanzeige und eine Skala (28) für die Achsenlage befindet und dass zur Beobachtung der Skalen das linke Okular (1b) des binokularen Tubus vorgesehen ist, dass weiter für die Dioptrieneinstellung und für die Hauptschnittlageneinstellung koaxial zueinander angeordnete Bedienelemente (5, 6) vorgesehen sind, und dass ein Hebel (7) mit drei Raststellungen für die Betätigung eines Blendenmechanismus für die Skalen- und Testfigurenbetrachtung vorgesehen ist.

2. Augenrefraktometer nach Anspruch 1, dadurch gekennzeichnet, dass die Bedienelemente (5, 6) und der Hebel (7) in doppelter Ausführung vorgesehen und zu beiden Seiten des Gerätes angebracht sind.

3. Augenrefraktometer nach Anspruch 1, dadurch gekennzeichnet, dass für die Messskalen (27, 28) eine digitale Anzeigevorrichtung vorgesehen ist.

**Revendications**

1. Réfractomètre oculaire pour la détermination objective de la réfraction de l'œil, comportant un système d'optomètre servant à former sur la rétine du sujet l'image d'un repère d'essai, ainsi qu'un système d'observation du repère d'essai et un système de visée pour aligner le réfractomètre sur l'œil du patient, caractérisé en ce que dans le trajet des rayons de formation d'image est prévu un objectif de formation d'image (22) pouvant coulisser verticalement et qui forme une image de la figure d'essai (2) dans un plan image intermédiaire (2'), qu'un prisme diviseur de rayonnement (23) est prévu pour la division géométrique des trajets de rayons de formation d'image et d'observation, qu'un objectif d'ophtalmoscope (24) commun aux trajets de rayons de formation d'image et d'observation est prévu et projette l'image intermédiaire (2') de la figure d'essai (2) sur la rétine (2'') du sujet et, en partant de celle-ci, forme à nouveau une image dans le plan image intermédiaire (2'), qu'un objectif d'observation (25) pouvant coulisser de façon synchrone de l'objectif de formation d'image (22) est prévu et forme, après réflexion sur le prisme diviseur de rayonnement (23), une image de l'image intermédiaire rétinienne (2') de la figure d'essai dans le plan de l'oculaire (1a) du tube de droite d'un tube binoculaire, que dans le système de visée est prévu un objectif de visée (26) qui forme dans le plan d'échelle (4) d'un télémètre une image de l'iris et de la pupille du sujet, que dans ce plan d'échelle se trouvent une échelle (27) pour l'indication des dioptries et une échelle (28) pour la position de l'axe et que pour observer les échelles est prévu l'oculaire gauche (1b) du tube binoculaire, qu'en outre pour le réglage des dioptries et pour le réglage de la position de la section principale sont prévus des éléments de manœuvre (5, 6) disposés coaxialement et qu'un levier (7) à trois positions d'arrêt est prévu pour l'actionnement d'un mécanisme de diaphragme pour l'observation de l'échelle et de la figure d'essai.

2. Réfractomètre selon la revendication 1, caractérisé en ce que les éléments de manœuvre (5, 6) et le levier (7) sont prévus en double et disposés des deux côtés de l'appareil.

3. Réfractomètre selon la revendication 1, caractérisé en ce que pour les échelles de mesure (27, 28) est prévu un dispositif d'affichage numérique.

**Claims**

1. Ophthalmic refractometer for objective refraction of the eye, with an optometer system for imaging a test target on the patient's retina, with an observation system for the test target and a sighting system for aligning the refractometer to the eye of the patient, characterized in that it has a vertically movable imaging objective lens (22) in the imaging beam path (30), which images the test target (2) in an intermediate image plane (2'), that it has a beamsplitting prism (23) for the geometrical splitting of the imaging and the observation beam paths, that it has a common ophthalmoscope objective lens (24) for the imaging and the observation beam paths, which projects the intermediate image (2') of the test target (2) onto the retina (2'') of the patient and images it from there back in the intermediate image plane (2'), that it has an observation objective lens (25) whose movement is synchronized with that of imaging objective lens (22), and which images the retinal intermediate image (2') of the test target, after reflection by the beam splitting prism (23), in the eyepiece plane (1a) of the right-hand half of a binocular tube, that it has a sighting objective lens (26) in the sighting system, which images the iris and the patient's pupil in the scale plane (4) of a distance measuring device, that it has a scale (27) for diopter display and a scale (28) for the axis position in this scale plane, and that the left-hand eyepiece (1b) of the binocular tube is used for viewing the scales, that furthermore it has controls (5, 6) arranged coaxially to each other for setting the diopter and the position of the principal meridian, and that it has a lever (7) with three detent positions for operating a diaphragm mechanism for observation of the scales and test target.

2. Ophthalmic refractometer according to claim 1, characterized in that the controls (5, 6) and the lever (7) are double and arranged on either side of the instrument.

3. Ophthalmic refractometer according to claim 1, characterized in that it has a digital display for measuring scales (27, 28).

1/2

# Fig.1

Fig. 2